(19) Europäisches Patentamt
European Patent Office
Office européen des brevets

(11) Numéro de publication: **0 278 846 B1**

(12) **FASCICULE DE BREVET EUROPEEN**

(45) Date de publication de fascicule du brevet: **15.04.92**

(51) Int. Cl.$^5$: **A61K 7/06**

(21) Numéro de dépôt: **88400204.9**

(22) Date de dépôt: **29.01.88**

# (54) Composition cosmétique utilisable sous forme vaporisée ou sous forme d'aérosol pour le soin, l'entretien et l'esthétique de la chevelure.

(30) Priorité: **29.01.87 FR 8701059**

(43) Date de publication de la demande:
**17.08.88 Bulletin 88/33**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:
**DE-A- 3 206 448**
**GB-A- 2 066 659**

**CHEMICAL ABSTRACTS, vol. 97, no. 26, décembre 1982, page 425, résumé no. 222747r, Columbus, Ohio, US; & JP-A-82 126 409 (KAO SOAP CO., LTD) 06-08-1982**

**CHEMICAL ABSTRACTS, vol. 94, no. 20, mai 1981, page 373, résumé no. 162601q, Columbus, Ohio, US; & JP-A-81 22 716 (UNILEVER N.V.) 03-03-1981**

(73) Titulaire: **LABORATOIRE LACHARTRE SOCIETE ANONYME:**
**104 Avenue du Général de Gaulle**
**F-92200 Neuilly sur Seine(FR)**

(72) Inventeur: **Scandel, Jean**
**1, Square Villaret de Joyeuse**
**F-75017 Paris(FR)**

(74) Mandataire: **L'Helgoualch, Jean**
**Cabinet Sueur et L'Helgoualch 78, rue Carnot**
**F-95240 Cormeilles-en-Parisis(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 278 846 B1

## Description

La présente invention concerne une nouvelle composition cosmétique pour le traitement des cheveux, et plus particulièrement une nouvelle composition cosmétique utilisable sous forme vaporisée ou sous forme d'aérosol, pour le soin, l'entretien et l'esthétique de la chevelure.

Les cheveux sont souvent dégradés sous l'action de l'environnement ou de divers traitements capillaires. Ainsi, certaines atmosphères polluées, des fumées, des salissures, ou des projections de divers produits, auxquelles sont parfois soumis les cheveux, ainsi qu'une exposition prolongée aux rayons ultraviolets, par exemple, entraînent une certaine dégradation de leur état. De même, l'action d'un brossage répété ou l'utilisation de certains produits capillaires tels que colorants, laques, fixateurs, etc, ou des opérations de séchage, d'ondulation, de décoloration, etc, destinées à modifier ou améliorer la présentation de la coiffure, peuvent altérer dans une certaine mesure les qualités des cheveux et leur être préjudiciables.

De telles dégradations se signalent souvent par des altérations de la fibre capillaire et/ou par des pointes fourchues des cheveux; elles peuvent aussi résulter d'une évolution naturelle ou d'un entretien inadapté. Ces dégradations donnent à la coiffure une apparence inesthétique, et on a donc envisagé diverses compositions cosmétiques et divers traitements capillaires pour éviter leur formation, les limiter ou les masquer.

Les compositions connues se présentent généralement sous forme de crèmes, gels, lotions ou aérosols, que l'on applique sur les cheveux et qui peuvent contenir une résine ou un agent filmogène dispersé ou dissous dans un solvant approprié, destiné à se fixer sur le cheveu et à former une gaine protectrice adhérant à sa fibre. Diverses résines ont été utilisées, et par exemple des éthers de cellulose comme dans le brevet US 3.876.760, des polyvinylpyrrolidones ou des copolymères vinyliques, et par exemple un copolymère d'éther vinylique et d'ester d'acide maléique, comme dans le brevet US 3.966.087, etc. Toutefois, ces compositions ont souvent l'inconvénient d'alourdir les cheveux, de provoquer une adhérence entre les fibres, ou encore de former un film discontinu sur le cheveu, et d'offrir une enduction alourdissante ainsi qu'une apparence médiocre de la coiffure. De plus, ces compositions ne permettent pas de prévenir ni de masquer les pointes fourchues.

Le brevet GB-A-2.066.659 décrit une composition de rinçage des cheveux, utilisable après un shampooing, contenant des sels d'ammonium quaternaire, un dérivé de silicone et un polyéthylène glycol. Le brevet DE-A-3.206.448 décrit également une composition de rinçage contenant un sel d'ammonium quaternaire, du dioxyde de silicium et un dérivé de silicone. Une autre composition de rinçage à base de sel d'ammonium quaternaire, de polymère cationique et de silicones est décrite dans la demande de brevet japonais 126409/82. Mais ces compositions de rinçage connues ne correspondant pas à l'objet de la présente invention. Par ailleurs, la demande de brevet japonais 22716/81 décrit l'utilisation de certains polysiloxanes comme conditionneurs des cheveux.

Il est donc souhaitable de pouvoir disposer d'une composition cosmétique pour le soin et l'entretien des cheveux qui soit facile à utiliser, qui les répare et les protège contre les altérations et/ou la formation de pointes fourchues, les limitant et les masquant lorsqu'elles existent, pour donner à la chevelure et à la coiffure une meilleure apparence esthétique.

Pour être efficace et facile à utiliser, une telle composition doit pouvoir être utilisable par vaporisation, et, de préférence, sous forme d'aérosol, assurant la pulvérisation de fines particules uniformément sur toute la chevelure. De plus, la composition ne doit pas bien entendu provoquer d'irritation du cuir chevelu, ni salir les cheveux par suite d'un dépôt excessif, ni laisser subsister une odeur ou une coloration désagréable. Enfin, les composants doivent être compatibles avec les supports utilisés, notamment les solvants, ainsi que les gaz propulseurs dans le cas des aérosols.

La présente invention a précisément pour objet une nouvelle composition liquide pour le soin, l'entretien et l'esthétique des cheveux, utilisable par vaporisation ou par brumisation, et plus particulièrement sous forme d'aérosol.

L'invention a également pour objet une nouvelle composition utilisable par vaporisation ou par brumisation procurant une micropulvérisation, et plus particulièrement sous forme d'aéro-sol, permettant d'éviter les altérations et/ou la formation de pointes fourchues des cheveux sous l'action de divers agents atmosphériques ou de traitements préjudiciables, ou de les limiter et de les masquer.

La présente invention a encore pour objet une nouvelle composition cosmétique utilisable par vaporisation ou par brumisation, et plus particulièrement en aérosol, ne présentant pas de propriété de fixation, améliorant instantanément l'aspect de la coiffure, en masquant les pointes fourchues et en évitant leur formation ultérieure éventuelle.

La composition cosmétique conforme à la présente invention comprend les composants suivants:

a - de 2,5 à 5% en poids environ de polysiloxane;

b - de 0,1 à 0,5% en poids environ d'un alcool gras;

c - de 0,05 à 0,5% en poids environ de squalane;

d - de 1 à 4% en poids environ d'un sel d'ammonium quaternaire;

e - de 0,1 à 0,5% en poids environ d'au moins un sel d'ammonium quaternaire ou au moins un sel de pyridinium quaternaire, comportant un ou deux groupes alkyles de 12 à 18 atomes de carbone;

dissous ou dispersés dans un solvant approprié.

En plus des composants indiqués ci-dessus, qui constituent les composants essentiels de la composition suivant la présente invention, divers additifs incorporés habituellement dans les compositions cosmétiques à usage capillaire peuvent également être utilisés, et par exemple, des parfums, des solubilisants pour parfums, des colorants, des conservateurs, etc.

Comme indiqué ci-dessus, la composition suivant l'invention est tout particulièrement destinée à une utilisation sous forme d'aérosol qui permet d'assurer une bonne distribution de fines particules de la composition sur toute la chevelure. La pressurisation est réalisée suivant les techniques connues. Tout dispositif procurant une pulvérisation fine et uniforme peut être utilisé.

Le gaz propulseur peut être par exemple un gaz sous pression tel que l'azote, ou encore un hydrocarbure volatil, tel que le butane, l'isobutane et le propane, et de préférence un hydrocarbure fluoré tel que le trichlorofluorométhane, le dichlorodifluorométhane, le chlorodifluorométhane, ou encore le dichlorotétrafluoroéthane. Ces hydrocarbures fluorés sont bien connus dans le commerce sous les marques Fréon 11, Fréon 12, Fréon 22 et Fréon 114, respectivement. Ils peuvent être utilisés isolément ou en mélange.

Le rapport en poids de la composition au gaz propulseur utilisé dans l'aérosol peut être avantageusement choisi entre 1:10 et 1:2 environ, et de préférence entre 1:5 et 1:3 environ, les valeurs précises étant déterminées en fonction des produits utilisés et des résultats recherchés, suivant les techniques classiques dans le domaine des aérosols.

Suivant une variante conforme à la présente invention, la composition peut être conditionnée pour être vaporisée directement sur les cheveux, au moyen de tout dispositif connu assurant une bonne micropulvérisation. On peut utiliser par exemple un dispositif à pompe ou un simple flacon à parois souples muni d'une buse de pulvérisation.

Le polysiloxane, l'alcool gras et le squalane utilisés dans la composition de l'invention exercent notamment une action de lubrifiant facilitant la mise en forme de la coiffure, tandis que le sel d'ammonium quaternaire et le sel d'ammonium ou de pyridinium quaternaire comportant une ou deux chaînes grasses, ont une action de conditionneur, assurant une amélioration de la présentation de la chevelure. Il est important que les rapports en poids indiqués ci-dessus soient respectés pour obtenir un résultat satisfaisant, c'est-à-dire pour masquer les pointes fourchues des cheveux, éviter leur formation ultérieure, et magnifier l'esthétique de la chevelure.

Le polysiloxane utilisé dans l'invention est de préférence un méthylpolysiloxane comportant des unités récurrentes de formule $-Si(CH_3)_2-O-$, qui a pour effet de réduire la friction entre les fibres kératiniques, ou entre les cheveux et les fibres de la brosse lors du brossage, ou les dents du peigne lors du peignage, et de faciliter la mise en forme de la coiffure. Le méthylpolysiloxane constituant le premier composant de la composition de l'invention est choisi parmi ceux solubles dans les solvants, tels que les alcools inférieurs, couramment utilisés pour les aérosols. On utilise de préférence un méthylpolysiloxane de poids moléculaire peu élevé, et par exemple un polyphénylméthylsiloxane tel que la phényl diméthicone.

L'alcool gras constituant le deuxième composant de la composition suivant l'inventicn représente entre 0,1 et 0,5% du poids total de la composition (non compris le gaz propulseur), et de préférence 0,2% en poids environ. On peut utiliser par exemple l'alcool cétylique, oléique, stéarylique, ou ricinoléique, et de préférence l'alcool ricinoléique de formule:

$$CH_3-(CH_2)_5-CHOH-CH_2-CH=CH-(CH_2)_7-CH_2OH$$

Conformément à l'invention, on a constaté que d'excellents résultats pouvaient être obtenus en incorporant à la composition une faible quantité de squalane, comprise entre 0,05 et 0,5% en poids environ, et de préférence entre 0,1 et 0,2% en poids.

Le squalane est un composé connu, représenté par la formule générale suivante:

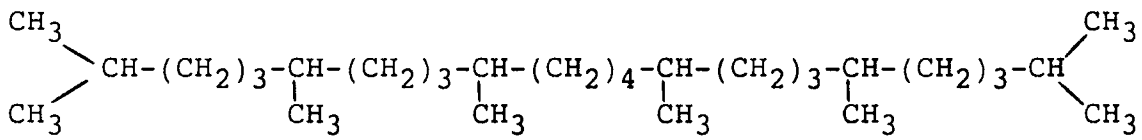

Comme indiqué ci-dessus, la composition suivant l'invention contient également un sel d'ammonium quaternaire, et par exemple un halogénure d'alkyl ammonium, tel qu'un bromure ou un chlorure, comportant ou non des groupes polaires, en une quantité comprise entre 1 et 4% en poids environ du poids total de la composition (non compris le gaz propulseur), et de préférence entre 1,5 et 2,5% en poids environ. On peut utiliser par exemple un chlorure dérivé d'une amine tertiaire polypropoxylée, tel que le composé connu sous la dénomination PPG-9 chlorure de diéthyl méthyl ammonium.

La composition suivant l'invention contient encore, comme constituant essentiel, au moins un sel, et de préférence un halogénure, d'ammonium quaternaire ou de pyridinium quaternaire comportant un ou deux groupes alkyles de 12 à 18 atomes de carbone, qui peut être choisi parmi les halogénures d'alkyl ammonium ou alkyl pyridinium quaternaire comportant au moins un groupe ayant entre 12 et 18 atomes de carbone dans la partie alkyle, et par exemple le chlorure de stéaryl pyridinium et le chlorure de lauryl pyridinium. Ces composés peuvent être utilisés isolément ou en combinaison. Ils représentent entre 0,1 et 0,5% en poids environ du poids total de la composition (non compris le gaz propulseur). On peut par exemple utiliser un mélange de chlorure de stéaryl pyridinium et de chlorure de lauryl pyridinium dans un rapport en poids égal à environ 3:1, ces deux composés représentant environ 0,2% du poids de la composition.

Le solvant utilisé pour la composition suivant l'invention est de préférence un alcool inférieur facilement volatil, du type utilisé dans les aérosols, tel que le méthanol, l'éthanol ou l'isopropanol, et de préférence l'éthanol.

Comme indiqué ci-dessus, la composition de l'invention peut encore contenir divers additifs usuels dans les compositions cosmétiques, et par exemple un parfum et un solubilisant du parfum.

La préparation de la composition s'effectue par simple mélange des composants décrits ci-dessus, et, dans le cas de l'utilisation sous forme d'aérosol, la mise sous pression dans un conteneur pour aérosol est réalisée par les techniques classiques en utilisant les gaz propulseurs précités.

La composition suivant l'invention peut être utilisée en aérosol, en la pulvérisant finement sur des cheveux préalablement coiffés, que l'on peigne à nouveau immédiatement après la pulvérisation. La durée de la pulvérisation, faite de façon continue, est généralement comprise entre 4 et 20 secondes, et de préférence entre 6 et 15 secondes, la quantité de composition et de gaz propulseur pulvérisée étant généralement comprise entre 2,5 et 10g. Cette vaporisation doit demeurer légère et ne pas entraîner le mouillage des cheveux, ni même leur humidification. Il est préférable de répéter la vaporisation, le cas échéant, plutôt que d'excéder le besoin des cheveux, dont dépend l'esthétique de la chevelure. Un effet d'amélioration de la présentation des cheveux est alors immédiatement obtenu.

La composition suivant l'invention peut également être utilisée dans un dispositif à pompe, ou dans un flacon en matière plastique déformable, sous forme de solution que l'on vaporise sur la chevelure préalablement coiffée, les cheveux étant à nouveau peignés immédiatement après l'application de la composition. Les quantités utilisées sont alors généralement comprises entre 1 et 3g suivant l'état de la chevelure.

Il est possible d'utiliser la composition suivant l'invention sans limitation particulière, ni de quantité, ni de fréquence, pour divers types de cheveux, mais de préférence sur des cheveux secs, des cheveux décolorés, des cheveux teints, des cheveux permanentés, ou des cheveux blancs, en évitant de l'utiliser sur des cheveux gras.

Les essais objectifs effectués en laboratoire ont montré que l'utilisation de la composition de l'invention entraîne une disparition statistiquement significative des pointes fourchues visibles, et une protection contre leur formation ultérieure. La mesure des forces de peignage par une technique dynamométrique connue a montré une amélioration significative du démêlage de la chevelure.

Les exemples ci-après illustrent l'invention plus en détail. Sauf indication contraire, les pourcentages, parties et rapports sont exprimés en poids.

**EXEMPLE 1**

On prépare une composition cosmétique en utilisant les composants indiqués ci-après:

| | |
|---|---|
| Phényl diméthicone | 4,00% |
| Alcool ricinoléique | 0,20% |
| squalane | 0,15% |
| PPG-9 chlorure de diéthyl méthyl ammonium, | 2,00% |
| Chlorure de stéaryl pyridinium | 0,15% |
| Chlorure de lauryl pyridinium | 0,05% |
| Parfum | 0,08% |

Solubilisant du parfum (Isosteareth 20) 0,05%
Ethanol dénaturé, pour compléter à 100,00%

Après mélange, la solution ci-dessus est mise en place dans un conteneur à valve pour aérosol, le gaz propulseur (mélange de Fréon 11 et de Fréon 12) est chargé, sous une pression de 1,5 bar environ.

On peigne une chevelure décolorée présentant des pointes fourchues et de nombreuses cassures de cheveux (environ 40% des cheveux), puis on procède à une pulvérisation de la composition ci-dessus; on peigne à nouveau les cheveux immédiatement après la pulvérisation afin de corréler l'action de la composition et le style de la coiffure.

On constate immédiatement une nette amélioration de l'aspect esthétique de la chevelure, et on observe aussi que les pointes fourchues des cheveux ne sont plus apparentes.

**EXEMPLE 2**

Une composition cosmétique conforme à la présente invention est préparée en utilisant les composants suivants, dans les proportions indiquées:
Phényl diméthicone 3,5%
Alcool cétylique 0,1%
Squalane 0,1%
PPG-9 chlorure de diéthyl méthyl ammonium 2,5%
Chlorure de stéaryl pyridinium 0,1%
Parfum 0,1%
Solubilisant du parfum 0,1%
Ethanol dénaturé, pour compléter à 100,0%

Les composants ci-dessus sont soigneusement mélangés, et la solution est placée dans un récipient pour aérosol, que l'on charge avec un gaz propulseur, comme dans l'exemple 1.

La composition pour aérosol ainsi préparée est utilisée dans les mêmes conditions que dans l'exemple 1, en effectuant une pulvérisation sur trois types de chevelures (cheveux légèrement secs, secs, très secs) présentant des pointes fourchues, à raison de 6 secondes pour la première (environ 3,g), 10 secondes pour la deuxième (environ 5,g), et 18 secondes pour la troisième (environ 8g).

Après coiffage au moyen d'un peigne, on observe dans les trois cas une amélioration immédiate des critères esthétiques de la chevelure. En outre, les pointes fourchues ne sont plus visibles à l'oeil nu.

**EXEMPLE 3**

On utilise la composition décrite dans l'exemple 1, sans la placer dans un récipient pour aérosol, mais dans un flacon de matière plastique (polyéthylène haute densité) de 100ml environ, muni d'un système à pompe mécanique permettant une micropulvérisation de la composition sur la chevelure préalablement coiffée.

La quantité utilisée pour une chevelure sèche d'importance moyenne est d'environ 2g.

Comme précédemment, les cheveux sont recoiffés immédiatement après l'application, et on observe une nette amélioration de l'état de la chevelure et de l'aspect de la coiffure, ainsi qu'une disparition des pointes fourchues visibles des cheveux.

**Revendications**

**1.** Composition cosmétique pour le soin, l'entretien et l'esthétique des cheveux, caractérisée en ce qu'elle comprend les composants suivants:
a - de 2,5 à 5% en poids de polysiloxane;
b - de 0,1 à 0,5% en poids d'un alcool gras;
c - de 0,05 à 0,5% en poids de squalane;
d - de 1 à 4% en poids d'un sel d'ammonium quaternaire;
e - de 0,1 à 0,5% en poids d'au moins un sel d'ammonium quaternaire différent du sel défini au paragraphe d) ci-dessus, ou au moins un sel de pyridinium quaternaire, comportant un ou deux groupes alkyles de 12 à 18 atomes de carbone;
dissous ou dispersés dans un solvant approprié.

**2.** Composition selon la revendication 1, caractérisée en ce que le polysiloxane est un méthylpolysiloxane.

**3.** Composition selon la revendication 1, caractérisée en ce que l'alcool gras est l'alcool ricinoléique ou l'alcool cétylique.

**4.** Composition selon la revendication 1, caractérisée en ce que le sel d'ammonium quaternaire est un halogénure d'alkyl ammonium.

**5.** Composition selon la revendication 1, caractérisée en ce que le sel d'ammonium quaternaire ou de pyridinium quaternaire comportant un ou deux groupes alkyles de 12 à 18 atomes de carbone, est un halogénure d'alkyl ammonium ou d'alkyl pyridinium, seul ou en mélange.

**6.** Composition selon la revendication 5, caractérisée en ce que le sel de pyridinium est un mélange de chlorure de stéaryl pyridinium et de chlorure de lauryl pyridinium dans un rapport en poids égal à 3:1.

**7.** Composition selon l'une quelconque des revendications précédentes, caractérisée en ce que le solvant est un alcool choisi parmi le méthanol, l'éthanol et l'isopropanol.

**8.** Composition cosmétique pour le soin, l'entretien et l'esthétique des cheveux, utilisable sous forme d'aérosol, caractérisée en ce que elle contient une composition selon l'une quelconque des revendications précédentes, en combinaison avec un gaz propulseur.

**9.** Composition selon la revendication 8, caractérisée en ce que le gaz propulseur est l'azote ou un hydrocarbure fluoré choisi parmi le trichlorofluorométhane, le dichlorodifluorométhane, le chlorodifluorométhane, et le dichlorotétrafluoroéthane.

**10.** Composition selon l'une quelconque des revendications 8 et 9, caractérisée en ce que le rapport en poids de la composition au gaz propulseur est compris entre 1:10 et 1:2.

**11.** Composition selon l'une quelconque des revendications 1 à 7, caractérisée en ce qu'elle est conditionnée dans un dispositif pour micropulvérisation.

## Claims

**1.** Cosmetic composition for the care, the maintenance and the esthetic of the hair, characterized in that it comprises the following components :

a - from 2.5 to 5.5% by weight of polysiloxane;
b - from 0.1 to 0.5% by weight of a fatty alcohol;
c - from 0.05 to 0.5% by weight of a squalane;
d - from 1 to 4% by weight of a quaternary ammonium salt;
e - from 0.1 to 0.5% by weight of at least one quaternary ammonium salt different from the salt defined in paragraph d) above, or at least one quaternary pyridinium salt, comprising one or more alkyl groups containing from 12 to 18 carbon atoms;

dissolved or dispersed in an appropriate solvent.

**2.** Composition according to claim 1, characterized in that said polysiloxane is a methylpolysiloxane.

**3.** Composition according to claim 1, characterized in that said fatty alcohol is ricinoleic alcohol or cetyl alcohol.

**4.** Composition according to claim 1, characterized in that said quaternary ammonium salt is an alkyl ammonium halide.

**5.** Composition according to claim 1, characterized in that said quaternary ammonium salt or said quaternary pyridinium salt comprising one or more alkyl groups having from 12 to 18 carbon atoms, is an alkyl ammonium halide or an alkyl pyridinium halide, alone or in admixture.

**6.** Composition according to claim 5, characterized in that said pyridinium salt is a mixture of stearyl pyridinium chloride and lauryl pyridinium chloride in a weight ratio of 3:1.

**7.** Composition according to any of the preceding claims, characterized in that said solvent is an alcohol selected from methyl alcohol, ethyl alcohol and isopropyl alcohol.

**8.** Cosmetic composition for the care, the maintenance and the esthetic of the hair, for use as an aerosol, characterized in that it contains a composition according to any of the preceding claims, in combination with a propellent.

**9.** Composition according to claim 8, characterized in that said propellent is nitrogen or a fluorinated hydrocarbon selected from trichlorofluoromethane, dichlorodifluoromethane, chlorodifluoromethane and dichlorotetrafluoroethane.

**10.** Composition according to any of claims 8 and 9, characterized in that the weight ratio of the composition to the propellent is from 1:10 to 1:2.

**11.** Composition according to any of claims 1 to 7, characterized in that it is conditioned in a device for microspraying.

**Patentansprüche**

**1.** Kosmetische Zusammensetzung für die Pflege, die Behandlung und die Ästhetik der Haare, dadurch gekennzeichnet, daß sie die folgenden Bestandteile umfaßt:
(a) 2,5 bis 5 Gew.-% Polysiloxan;
(b) 0,1 bis 0,5 Gew.-% eines Fettalkohols;
(c) 0,05 bis 0,5 Gew.-% Squalan;
(d) 1 bis 4 Gew.-% eines quaternären Ammoniumsalzes; und
(e) 0,1 bis 0,5 Gew.-% mindestens eines quaternären Ammoniumsalzes, das von dem oben unter (d) definierten Salz verschieden ist, oder mindestens eines quaternären Pyridiniumsalzes, das eine oder zwei Alkylgruppen mit 12 bis 18 Kohlenstoffatomen trägt;
die in einem geeigneten Lösungsmittel gelöst oder dispergiert sind.

**2.** Zusammensetzung nach Anspruch 1, dadurch gekennzeichnet, daß es sich bei dem Polysiloxan um ein Methylpolysiloxan handelt.

**3.** Zusammensetzung nach Anspruch 1, dadurch gekennzeichnet, daß es sich bei dem Fettalkohol um den Phizinolalkohol oder den Cetylalkohol handelt.

**4.** Zusammensetzung nach Anspruch 1, dadurch gekennzeichnet, daß es sich bei dem quaternären Ammoniumsalz um ein Alkylammoniumhalogenid handelt.

**5.** Zusammensetzung nach Anspruch 1, dadurch gekennzeichnet, daß es sich bei dem quaternären Ammoniumsalz oder dem quaternären Pyridiniumsalz, das ein oder zwei Alkylgruppen mit 12 bis 18 Kohlenstoffatomen trägt, um ein Alkylammoniumhalogenid oder ein Alkylpyridininiumhalogenid, allein oder im Gemisch, handelt.

**6.** Zusammensetzung nach Anspruch 5, dadurch gekennzeichnet, daß es sich bei dem Pyridiniumsalz um ein Gemisch von Stearylpyridiniumchlorid und Laurylpyridiniumchlorid in einem Gewichtsverhältnis von 3:1 handelt.

**7.** Zusammensetzung nach einem der vorhergehenden Ansprüche, dadurch gekennzeichnet, daß es sich bei dem Lösungsmittel um einen Alkohol, ausgewählt aus Methanol, Ethanol und Isopropanol, handelt.

**8.** Kosmetische Zusammensetzung für die Pflege, die Behandlung und die Ästhetik der Haare, die in Form eines Aerosols verwendbar ist, dadurch gekennzeichnet, daß sie eine Zusammensetzung nach einem der vorhergehenden Ansprüche in Kombination mit einem Treibgas enthält.

**9.** Zusammensetzung nach Anspruch 8, dadurch gekennzeichnet, daß es sich bei dem Treibgas um Stickstoff oder einen fluorierten Kohlenwasserstoff, ausgewählt aus Trichlorofluoromethan, Dichlorodifluoromethan, Chlorodifluoromethan und Dichlorotetrafluoroethan, handelt.

**10.** Zusammensetzung nach einem der Ansprüche 8 und 9, dadurch gekennzeichnet, daß das Gewichtsverhältnis zwischen der Zusammensetzung und dem Treibgas zwischen 1:10 und 1:2 liegt.

**11.** Zusammensetzung nach einem der Ansprüche 1 bis 7, dadurch gekennzeichnet, daß sie in einer Feinmahlvorrichtung konditioniert worden ist.